# EUROPÄISCHE PATENTSCHRIFT

(11) **EP 3 229 878 B1**
(45) Veröffentlichungstag und Bekanntmachung des Hinweises auf die Patenterteilung: **23.01.2019**
(21) Anmeldenummer: 15808367.5
(22) Anmeldetag: 04.12.2015
(51) Int. Cl.: A61M 15/00, B65D 47/08

(54) **HANDGERÄT ZUR AUSGABE UND INHALATION EINER PHARMAZEUTISCHEN SUBSTANZ**
HANDHELD DEVICE FOR DISPENSING AND INHALATION OF A PHARMACEUTICAL SUBSTANCE
APPAREIL PORTATIF POUR DÉLIVRER ET INHALATION D'UNE SUBSTANCE PHARMACEUTIQUE

(30) Priorität: 09.12.2014 DE 102014118248
(43) Veröffentlichungstag der Anmeldung: 18.10.2017
(73) Patentinhaber: Von Schuckmann, Alfred, 47627 Kevelaer (DE)
(72) Erfinder: Von Schuckmann, Alfred, 47627 Kevelaer (DE)
(74) Vertreter: Müller, Enno
(86) Internationale Anmeldenummer: PCT/EP2015/078624
(87) Internationale Veröffentlichungsnummer: WO 2016/091737

(56) Entgegenhaltungen:
- EP-A1- 1 147 054
- EP-A1- 1 696 987
- GB-A- 2 294 506
- US-A- 2 620 061
- US-A1- 2008 035 681
- US-A1- 2010 065 589
- US-A1- 2013 213 394

## Beschreibung

Die Erfindung betrifft ein Handgerät nach den Merkmalen des Oberbegriffes des Anspruches 1.

Handgeräte der in Rede stehenden Art sind bekannt. Diese dienen beispielsweise zur Inhalation pharmazeutischer Substanzen, so beispielsweise zur Inhalation pulverförmiger Präparate oder zur Inhalation von pharmazeutische Substanzen enthaltenden Aerosolen.

Das Handgerät nimmt beispielsweise einen Behälter für das Aerosol auf oder formt eine Aufnahme eines Trägers, beispielsweise ein Blister oder eine Kapsel zur Pulverinhalation. Das Handgerät weist ein Mundstück auf, welches zur Inhalation der Substanz von den Lippen des Benutzers umschlossen wird.

Bekannt ist diesbezüglich auch, das Mundstück bei Nichtbenutzung des Handgerätes mit einer Kappe zu verschließen. In diesem Zusammenhang besteht das Bedürfnis, die Kappe unverlierbar an dem Handgerät anzubinden. Es sind diesbezüglich unterschiedliche Lösungen bekannt. Eine Federanbindung und die Freigabe des Mundstückes durch Verschwenken der Kappe um eine Schwenkachse ist beispielsweise aus der DE 697 23 156 T2 (US 5,896,853 A) bekannt. Die Feder ist durch ein filmscharnierartiges Gelenk gebildet. Aus der US 2 620 061 A ist eine um eine Gelenkachse drehbare Kappe an einem Gehäuse bekannt, wobei die Kappe mittels einer Wendelfeder, durch welche hindurch sich die Achse erstreckt, beaufschlagt ist. Aus der GB A 2 294 506 ist ein Handgerät mit einem Mundstück und einer dieses verschließenden Kappe bekannt, bei welcher die Kappe mittels eines Bandelementes, das sich im Verschlusszustand der Kappe in einer Einfaltelung befindet, an dem Gehäuse angebunden ist. In einer Draufsicht, in welcher die Schwenkachse des Bandelementes sich als Linie abbildet, weist dieses einen geraden Verlauf auf. Aus der WO 2005/046774 A1 ist ein Handgerät mit einem Mundstück und einer dieses verschließenden Kappe bekannt, wobei die Kappe verschiebbar, aber unverlierbar an einem mit dem Gehäuse verbundenen Bandelement angebracht ist.

Im Weiteren ist zum Stand der Technik auf die EP 1 696 987 A1, die US 2013/213394 A1, die EP 1147 054 A1, die US 2010/035681 A1 und die US 2008/ 035681 A1 zu verweisen.

Ausgehend von dem zunächst genannten Stand der Technik stellt sich der Erfindung die Aufgabe, eine vorteilhafte Ausgestaltung eines Handgerätes mit einer ein Mundstück verschließenden Kappe anzugeben.

Diese Aufgabe ist beim Gegenstand des Anspruches 1 gelöst, wobei darauf abgestellt ist, dass die Kappe entgegen einer Vorspannung der Feder in der Öffnungsrichtung verlagerbar ist, dass die Feder zwei Federarme aufweist, die an zwei quer zu der Öffnungsrichtung distanzierten Befestigungsstellen mit der Kappe verbunden sind, dass die Federarme gehäuseseitig in einem gemeinsamen Anbindungsbereich, der mit dem Gehäuse über ein Gelenk verschwenkbar verbunden ist, zusammengeführt sind und dass die Kappe mitsamt dem Anbindungsbereich nach unten unter einen Gehäuseboden abschwenkbar ist..

Der abgewinkelte Verlauf stellt sich zumindest in einer Federstellung ein. Bevorzugt stellt sich der abgewinkelte Verlauf zumindest in der entspannten Federstellung ein, wie auch in einer zur Abnahme der Kappe von dem Mundstück üblichen Federspannstellung. Auch kann der abgewinkelte Verlauf in jeder Federstellung zwischen der entspannten Stellung und der im üblichen Gebrauch maximal gespannten Stellung vorliegen.

Die Feder weist - bezogen auf die Draufsicht - in ihrem Verlauf zumindest einen Abschnitt auf, der zu einem weiteren, anschließenden Federabschnitt einen Winkel von weniger als 180° einschließt.

Durch den abgewinkelten Verlauf kann die Federwirkung verbessert sein. Darüber hinaus ist eine Verlängerung des wirksamen Federweges hierdurch erreichbar.

Die Feder weist zwei Federarme auf, die an zwei quer zu der Öffnungsrichtung distanzierten Befestigungsstellen mit der Kappe verbunden sind.

Durch die Anordnung zweier, in Öffnungsrichtung zueinander distanzierter Federarme ist eine stabile federbare Anbindung der Kappe an das Gehäuse erreichbar.

Beide Federarme können in einer Draufsicht, in welcher sich die Schwenkachse als Linie abbildet, einen abgewinkelten Verlauf aufweisen.

Der abgewinkelte Verlauf eines Federarms kann mit Bezug auf die Draufsicht spiegelbildlich zum abgewinkelten Verlauf des anderen Federarmes ausgebildet sein.

Dadurch, dass die Feder beziehungsweise ein Federarm in einem entspannten Zustand über die Länge betrachtet teilweise mit einer Richtungskomponente entgegen der Öffnungsrichtung verläuft, ist die Feder beziehungsweise ein Federarm zumindest partiell mit einem Abschnitt versehen, der eine Richtungskomponente entgegen der Abzugsrichtung der Kappe vom Mundstück beziehungsweise eine Richtungskomponente in Aufsteckrichtung der Kappe auf das Mundstück aufweist. Dies bietet das genannte Längenreservoir für die Feder beziehungsweise für den Federarm beim Spannen der Feder im Zuge eines Abzugsvorganges der Kappe von dem Mundstück.

Bevorzugt resultiert die Längenänderung der Feder beziehungsweise des Federarmes zwischen der Kappe und der Anbindung der Feder an dem Gehäuse bei eine Verlagerung der Kappe in Öffnungsrichtung nicht oder nicht wesentlich aus einer gegebenen Elastizität des Federmaterials, sondern vielmehr durch eine Zugabe von Federlänge aus einem Reservoirabschnitt der Feder. Dies kann gegebenenfalls so weit führen, dass in einer extremen Spannsituation der Kappe die Feder beziehungsweise der Federarm zwischen der Anbindung an der Kappe und der Anbindungsstelle an dem Gehäuse beziehungsweise an einem dem Gehäuse zugeordneten Abschnitt zumindest annähernd langgestreckt linear verläuft, unter Nutzung des gesamten Längenreservoirs.

Die Federarme sind, abgewandt der Anbindung an der Kappe, gehäuseseitig in einem gemeinsamen Anbindungsbereich zusammengeführt. Der Anbindungsbereich ist bevorzugt ein starrer, d.h. nicht federbarer Abschnitt.

Der Anbindungsbereich kann unmittelbar ein Gehäuseabschnitt sein. Auch kann der Anbindungsbereich ein dem Gehäuse zuordbarer Abschnitt sein, so weiter beispielsweise ein integraler Abschnitt eines Federelementes.

Der Anbindungsbereich ist mit dem Gehäuse über ein Gelenk verschwenkbar verbunden. Die diesbezügliche Schwenkachse verläuft bevorzugt quergerichtet zu der Öffnungsrichtung der Kappe. Auch kann sich die Schwenkachse, insbesondere die geometrische Schwenkachse in einer durch die Feder beziehungsweise durch die Federarme aufgespannten Ebene erstrecken.

Weiter können die Federarme sich in einer Fläche erstrecken, die im Verschlusszustand, d.h. bei auf dem Mundstück aufgesetzter Kappe, angepasst an eine Zuordnungsfläche des Gehäuses verläuft. Die Erstreckungsfläche der Federarme verläuft bevorzugt parallel zu einer zugeordneten Fläche des Gehäuses. Auch können sich die Federarme der Feder zumindest partiell auf dieser Zuordnungsfläche des Gehäuses abstützen.

Die Kappe kann in der Verschlussstellung mit Vorspannung an dem Gehäuse anliegen. So liegen die Federarme in der Mundstück-Verschlussstellung bevorzugt nicht in ihrem entspannten Zustand vor, sondern vielmehr unter Beibehaltung einer Spannung. Dies unterstützt die Verschlussposition der Kappe.

Zum Abnehmen der Kappe ist diese gegen die Kraft der Feder beziehungsweise der Federarme in Öffnungsrichtung zu bewegen.

Der Anbindungsbereich kann gehäuseseitig eine Achsausformung aufweisen. Diese Achsausformung ist geeignet zum Verschwenken des Anbindungsbereiches und somit bevorzugt der gesamten Feder mit der Kappe relativ zu dem Gehäuse. Auch kann der Anbindungsbereich und hiermit auch die Feder mit der angebundenen Kappe dem Gehäuse steckzuordbar sein. Hierzu kann das Gehäuse eine Einklips-Ausformung für die Achsausformung des Anbindungsbereiches aufweisen. Die Einklips-Ausformung kann so ausgelegt sein, dass eine herbeigeführte Klips-Verbindung zwischen Gehäuse und Anbindungsbereich nicht oder nicht werkzeuglos wieder aufhebbar ist.

Die Feder kann in der Draufsicht W-förmig gestaltet sein, wobei ein W-Mittelbereich durch den Anbindungsbereich (mit)gegeben ist. Die äußeren W-Schenkel formen im Wesentlichen die Federarme aus. Die im Vergleich hierzu betrachteten inneren W-Schenkel können die teilweise mit einer Richtungskomponente entgegen der Öffnungsrichtung verlaufenden Federarmabschnitte sein. Diese Federarmabschnitte sind bevorzugt an dem Anbindungsbereich befestigt.

Die Feder beziehungsweise ein Federarm weist zumindest eine von einer Anbindungsstelle an dem Gehäuse bis zu der Kappe reichende Länge auf, wobei Abschnitte der Feder durchaus auch nicht federnd sein können (beispielsweise der Anbindungsbereich). Weiter kann die Feder in der Verschlussstellung von einer Anbindungsstelle an dem Gehäuse bis zu einer Anbindungsstelle an der Kappe eine größere Erstreckung aufweisen als in einem entspannten Zustand, jedoch eine kleinere Erstreckung als im Zuge eines Abziehens der Kappe von dem Mundstück.

Ausgehend von dem Anbindungsbereich kann ein Federarm so ausgebildet sein, dass sich dieser einschwenkend um eine senkrecht zu der Öffnungssrichtung beziehungsweise senkrecht zu der Draufsicht verlaufenden Schwenkachse bewegen kann.

Auch kann die Feder beziehungsweise können die Federarme sich allein nur in der durch die Federarme aufgespannten Ebene bewegen, also bevorzugt sowohl im Zuge der Abzugsbewegung der Kappe vom Mundstück als auch beim Aufsetzen der Kappe. So verläuft die Feder beziehungsweise verlaufen die Federarme bevorzugt sowohl im gespannten als auch im entspannten Zustand in derselben Ebene, in welcher Ebene weiter bevorzugt sich auch der Anbindungsbereich erstreckt.

Auch kann sich in der durch die Feder und bevorzugt auch durch den Anbindungsbereich aufgespannten Ebene ein Wandungsabschnitt der Kappe erstrecken, bevorzugt ein insbesondere die Feder umgreifender Längswandungsabschnitt derselben.

Nachstehend ist die Erfindung anhand der beigefügten Zeichnung, welche lediglich ein Ausführungsbeispiel darstellt, näher erläutert. Es zeigt:
- Fig. 1: in einem Längsschnitt ein Handgerät zur Ausgabe einer pharmazeutischen Substanz mit einem durch eine Kappe verschlossenen Mundstück;
- Fig. 2: eine der Figur 1 entsprechende Darstellung, betreffend eine Zwischenstellung im Zuge einer Entfernung der Kappe vom Mundstück;
- Fig. 3: eine Folgedarstellung zur Figur 2 bei freigegebenem Mundstück;

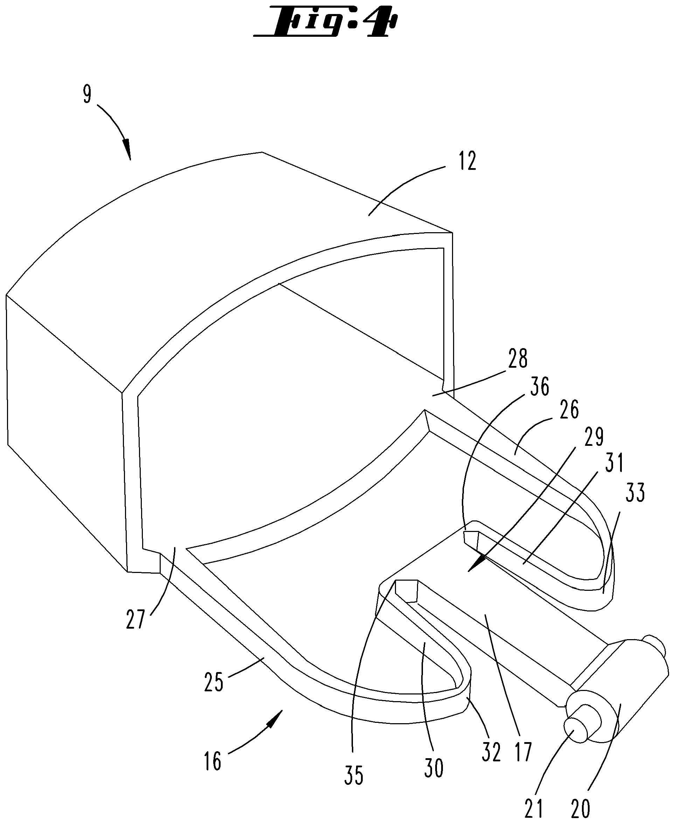

- Fig. 4: in perspektivischer Einzeldarstellung die Kappe mit einer angeformten Feder;
- Fig. 5: die Unteransicht gegen das Gerät in einer Mundstück-Verschlussstellung gemäß Figur 1;
- Fig. 6: den vergrößerten Schnitt gemäß der Linie VI-VI in Figur 5.

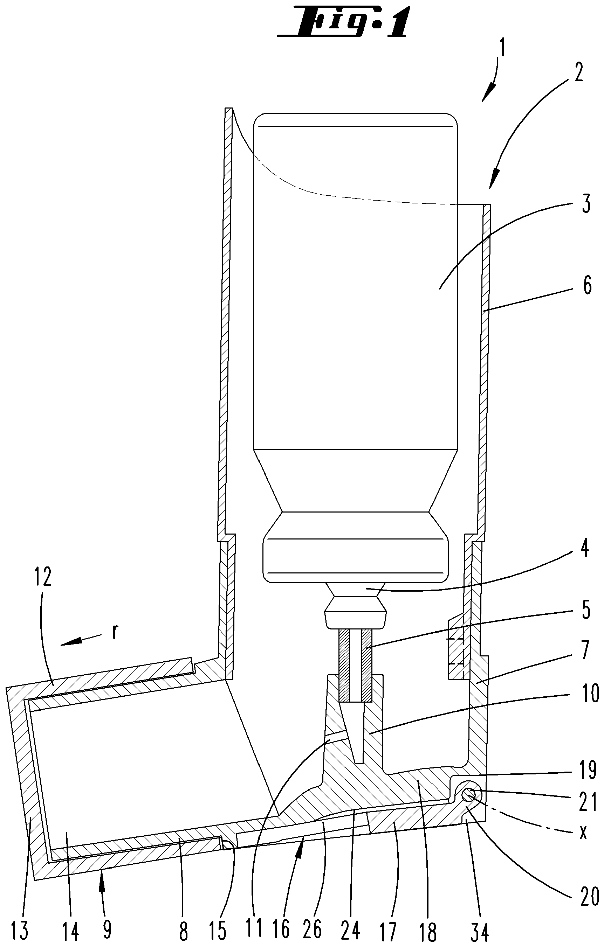

Dargestellt und beschrieben ist, zunächst mit Bezug zu Figur 1, ein Handgerät 1 zur Ausgabe einer pharmazeutischen Substanz.

Das Handgerät 1 weist ein Gehäuse 2 auf, in welches eine die sprühfähige Substanz beinhaltende Kartusche 3 einsetzbar ist. Diese Kartusche 3 ist in dem Gehäuse 2 axial verschiebbar.

In üblicher Weise weist der Kartuschenkopf 4 ein zentrales, sich koaxial zur Kartusche 3 erstreckendes Ventilrohr 5 auf. Über letzteres wird eine Medikamentenausgabe durch eine axiale Relativbewegung zwischen Kartusche 3 und Gehäuse 2 erreicht.

Das Gehäuse 2 kann zweigeteilt sein und im Wesentlichen aus zwei übereinander angeordneten Ringteilen 6 und 7 bestehen, von welchen das obere Ringteil 6 schaftförmig ausgeformt ist und das untere Ringteil 7 ein etwa quer zur Schafterstreckung ausgerichtetes Mundstück 8 aufweist. Letzteres ist durch eine Kappe 9 verschließbar.

Das Ventilrohr 5 der Kartusche 3 stützt sich in einem zugeordneten rohrförmigen Stützabschnitt 10 innerhalb des unteren Ringteils 7 ab, dies bei axialer Beweglichkeit der Kartusche 3 innerhalb des die Kartusche 3 umgebenden, schaftartigen Ringteils 6.

Der das Ventilrohr 5 der Kartusche 3 klemmend aufnehmende, innerhalb des unteren Gehäuse-Ringteils 7 ausgeformte Stützabschnitt 10 ist mit einem gegenüber einem das Ventilrohrende aufnehmenden Abschnitt durchmesserverringerten Strömungskanal 11 versehen, welcher strömungstechnisch in Verbindung steht mit dem Ventilrohr 5, wobei das dem Ventilrohr 5 abgewandte Ende des Strömungskanals 11 in Richtung auf das Mundstück 8 weist.

Die beiden Ringteile 6 und 7 sind in dem dargestellten Ausführungsbeispiel steckbar miteinander verbunden. Alternativ können die beiden Teile auch über ein Gewinde, beispielsweise über ein Grobgewinde mit hoher Steigung, miteinander verbunden sein.

Das zweiteilige Gehäuse 2 wie auch die Kappe 9 sind bevorzugt als KunststoffSpritzteile hergestellt.

In der Verschlussstellung überfängt die im Wesentlichen topfförmige Kappe 9 das rohrartige Mundstück 8 derart, dass die, die Topfwandung bildende, umlaufende Wandung 12 der Kappe 9 das Mundstück 8 umfasst und eine topfbodenartige Kappendecke 13 die Mundstücköffnung 14 überfängt.

Die auf das Mundstück 8 aufgesteckte Kappe 9 tritt anschlagbegrenzend gegen eine wurzelseitig des Mundstücks 8 ausgeformte, bevorzugt umlaufende Stufe 15 des Ringteiles 7.

Die Kappen-Aufsteckstellung gemäß Figur 1 ist bevorzugt rastgesichert, wozu nicht dargestellte Rastmittel und Gegenrastmittel Kappen- und mundstückseitig vorgesehen sind.

Die Kappe 9 ist unverlierbar an dem Gehäuse 2 beziehungsweise an dem Ringteil 7 gehaltert.

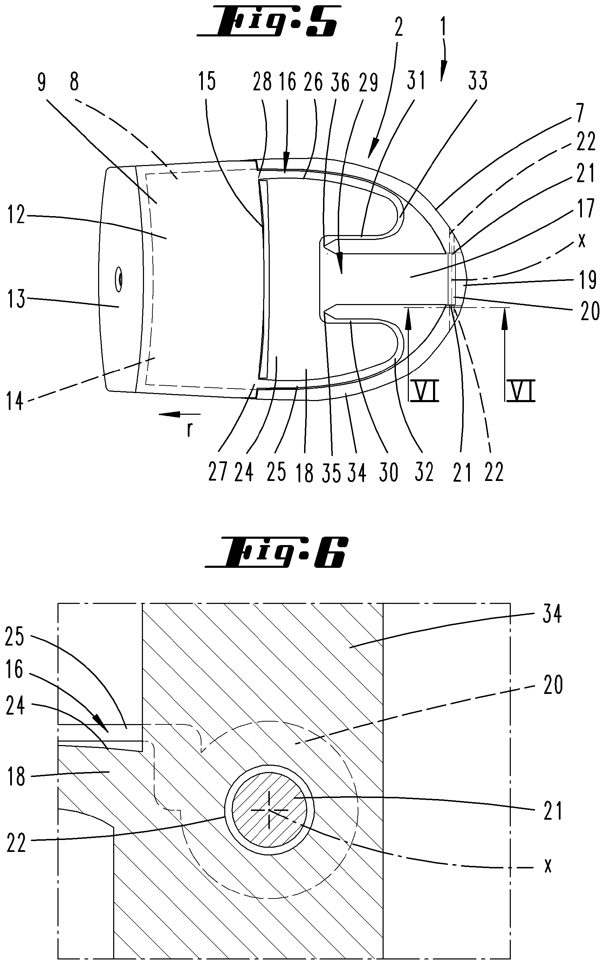

Hierzu ist an der Kappe 9 eine Feder 16 angebunden. Diese ist bevorzugt einstückig und materialeinheitlich mit der Kappe 9 ausgebildet. Die Feder 16 verläuft in einer Draufsicht, vgl. etwa Figur 5, in welcher sich eine Schwenkachse x, um welche die Kappe 9 zur Freigabe des Mundstücks 8 verschwenkbar ist, als Linie abbildet, mit einem abgewinkelten Verlauf. In der Ansicht der Figur 5, also der genannten Draufsicht, verläuft die Schwenkachse in der Zeichenebene quasi als Endloslinie, von welcher aber nur ein Abschnitt zeichnerisch dargestellt ist.

Die Feder 16 geht der Kappe 9 abgewandt in einen gleichfalls bevorzugt einstückig und materialeinheitlich mit der Feder 16 und Kappe 9 ausgebildeten Anbindungsbereich 17 über.

Über den Anbindungsbereich 17 ist die Kappe 9 insgesamt zusammen mit der Feder 16 und dem Anbindungsbereich 17 schwenkbeweglich an dem Gehäuse 2, insbesondere an dem Ringteil 7 befestigt.

Die schwenkbare Festlegung ist bodenseitig des Ringteils 7 vorgesehen, weiter insbesondere in einem - mit Bezug auf eine Längsschnittdarstellung gemäß Figur 1 - Eck-Übergangsbereich vom Gehäuseboden 18 in die umlaufende Wandung des Ringteils 7.

In diesem Eckbereich ist das Ringteil 7 mit einer wannenartigen Vertiefung 19 versehen. In dieser liegt ein insgesamt im Wesentlichen kreiszylinderartiger Endabschnitt 20 des Anbindungsbereiches 17 ein.

Die resultierende geometrische Schwenkachse x ist quergerichtet zu einer zentralen Körperachse des, die Kartusche 3 umfassenden Ringteils 6.

Es ist eine Achse 21 vorgesehen, die von der Schwenkachse x zentral in Längsrichtung der Achse 21 durchsetzt ist. Die Achse 21 axial über einen oder beide Endabschnitte 20 hinaus und greift in entsprechend positionierte Bohrungen 22 im Ringteil 6.

Der Anbindungsbereich 17 ist als plattenförmiges Starrelement ausgebildet, mit einer in der Mundstück-Verschlussstellung gemäß Figur 1 zumindest annähernd zur Zuordnungsfläche 24 des Gehäusebodens 18 parallel verlaufenden Breitfläche, welche einem Mehrfachen, beispielsweise einem Sieben- bis Fünfzehn-Fachen der senkrecht hierzu betrachteten Materialstärke des Anbindungsbereiches 17 entspricht.

Ausgehend von dem Endabschnitt 20 erstreckt sich der Anbindungsbereich 17 mit Bezug auf die in Figur 1 dargestellte Mundstück-Verschlussstellung etwa über die Hälfte der sich zwischen Endabschnitt 20 und der zugewandten Randkante der Kappe 9 ergebenden kürzesten Strecke.

Weiter erstreckt sich der Anbindungsbereich 17 bevorzugt zumindest annähernd in einer gemeinsamen Fläche mit einem unterseitigen Wandungsabschnitt der Kappenwandung 12.

Auch die Feder 16 erstreckt sich bevorzugt und zumindest in der Mundstück-Verschlussstellung in dieser gemeinsamen Fläche.

Die Feder 16 weist zwei Federarme 25, 26 auf. Diese sind einerends an zwei quer zu einer Öffnungsrichtung r der Kappe 9 zueinander distanzierten Befestigungsstellen 27, 28 mit der Kappe 9 verbunden. Die Befestigungsstellen 27, 28 sind bevorzugt stirnseitig des Öffnungsrandes der Kappe 9, insbesondere an dem Randabschnitt der das Mundstück 8 unterfangenden Wandung ausgebildet.

Der Abstand der Befestigungsstellen 27 und 28 zueinander kann angepasst sein an das in selber Richtung betrachtete innere Öffnungsmaß der Kappe 9.

Den Befestigungsstellen 27, 28 abgewandt sind die Federarme 25 und 26 in dem, dem Endabschnitt 20 abgewandten freien Endbereich an dem Anbindungsbereich 17 befestigt.

Der Abstand der Befestigungspunkte am Anbindungsbereich 17 entspricht etwa einem Drittel bis der Hälfte des Abstandes der Befestigungsstellen 27, 28 an der Kappe 9.

Die Feder 16 weist insgesamt mit Bezug auf eine Draufsicht gemäß Figur 5, in welcher sich die Schwenkachse x als Linie zeigt, eine W-förmige Gestalt auf. In dem sich hierbei ergebenden W-Mittelbereich 29 ist die Anbindung an den Anbindungsbereich 17 gegeben.

Hieraus ergibt sich je Federarm 25, 26 zumindest im entspannten Zustand der Feder 16, darüber hinaus aber auch in der Mundstück-Verschlussstellung gemäß Figur 5, jeweils ein Federarmabschnitt 30, 31, welcher mit einer Richtungskomponente entgegen der Öffnungsrichtung r verläuft.

Jeder Federarm 25, 26 erstreckt sich ausgehend von der Kappe 9 mit Bezug auf einen Grundriss gemäß Figur 5 über den freien Endbereich des Anbindungsbereiches 17 in Richtung auf den Endabschnitt 20 hinaus, geht hier über in einen kreisringförmig verlaufenden Umkehrabschnitt 32, 33, an welchem sich der an dem Anbindungsbereich 17 angebundene Federarmabschnitt 30, 31 anschließt.

Die Federarmabschnitte 30 und 31 sind jeweils über ein Filmscharnier 35, 36 an dem Anbindungsbereich 17 angelenkt. Im Bereich der Filmscharniere 35, 36 ergibt sich eine Dünnstelle (Materialverjüngung) mit einer bevorzugten Materialstärke von 0,05 bis 0,5 mm, insbesondere 0,1 bis 0,4 mm.

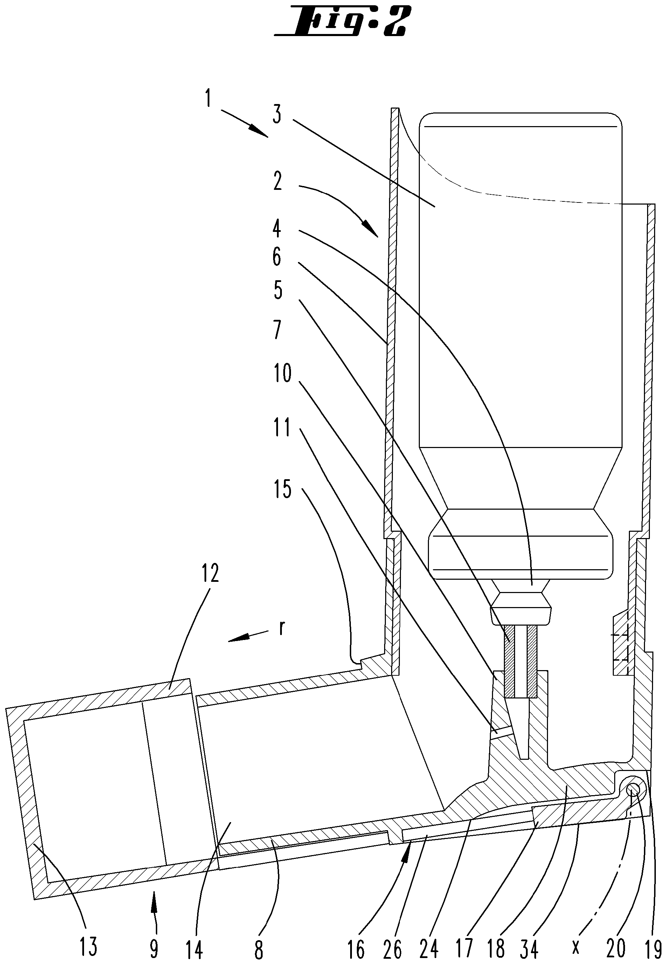

Der Umkehrabschnitt 32, 33 mit dem sich anschließenden Federarmabschnitt 30, 31 bietet ein Federarm-Reservoir, zur Verlängerung der Federarme 25, 26 im Zuge einer Abzugsbewegung der Kappe 9 von dem Mundstück 8 (vergleiche Figur 2).

Die Federarme 25 und 26 sind stegartig gebildet, mit einem bevorzugten rechteckigen Querschnitt. So ist weiter bevorzugt im Bereich der äußeren W-Schenkel ein zumindest annähernder quadratischer Querschnitt vorgesehen, beispielsweise mit einer jeweiligen Kantenlänge von 1,3 bis 2 mm, weiter beispielsweise 1,6 mm.

In den Umkehrbereichen 32 und 33 hingegen ist bevorzugt ein langgestreckt rechteckiger Querschnitt vorgesehen, mit einer senkrecht zur Erstreckungsfläche der Feder 16 betrachteten Höhe, die etwa einem 3- bis 4-Fachen der quer hierzu betrachteten Materialstärke im Umkehrbereich 32, 33 entspricht. So ist beispielsweise eine Höhe von 2 bis 3 mm, bevorzugt etwa 2,5 mm vorgesehen und eine Materialstärke von 0,5 bis 1 mm, beispielsweise 0,7 mm.

Insbesondere die Querschnittsgestaltung im Umkehrbereich 32, 33 erlaubt eine günstige Umlenkung und Federarmverschiebung bei einer Verlagerung der Kappe 9 entgegen der Federkraft.

Insgesamt weist die Feder 16, insbesondere jeder Federarm 25, 26 einen in der Draufsicht gemäß Figur 5 abgewinkelten Verlauf auf, weiter insbesondere bezüglich der äußeren W-Abschnitte der Feder 16 zu den inneren W-Schenkeln in Form der Federarmabschnitte 30, 31.

Beide Federarme 25 und 26, wie insgesamt die Feder 16, erstrecken sich in einer gemeinsamen Fläche, die im Mundstück-Verschlusszustand gemäß Figur 1 angepasst an die Zuordnungsfläche 24 des Gehäuses 2 verläuft, gegebenenfalls sich hierauf abstützend.

Die Feder 16 und der Anbindungsbereich 17 sind weiter bevorzugt umfasst von einem sich über die Zuordnungsfläche 24 des Bodens 18 hinaus erstreckenden Gehäusewandabschnitt 34, womit sich insbesondere in der Mundstück-Verschlussstellung die Feder 16 und der Anbindungsbereich 17 in einer gehäusebodenseitigen Senke erstrecken.

Zur Freilegung des Mundstückes 8 ist die Kappe 9 entgegen der Vorspannung der Feder 16, welche bevorzugt auch in der Mundstück-Verschlussstellung auf die Kappe 9 wirkt, in Öffnungsrichtung r zu verlagern. Hierbei handelt es sich im Wesentlichen um eine Linearverlagerung der Kappe 9 relativ zu dem Mundstück 8.

Die Feder 16 wird hierbei weiter gespannt, dies unter quasi abrollender Umlenkung und Verlängerung der Federarme 25, 26.

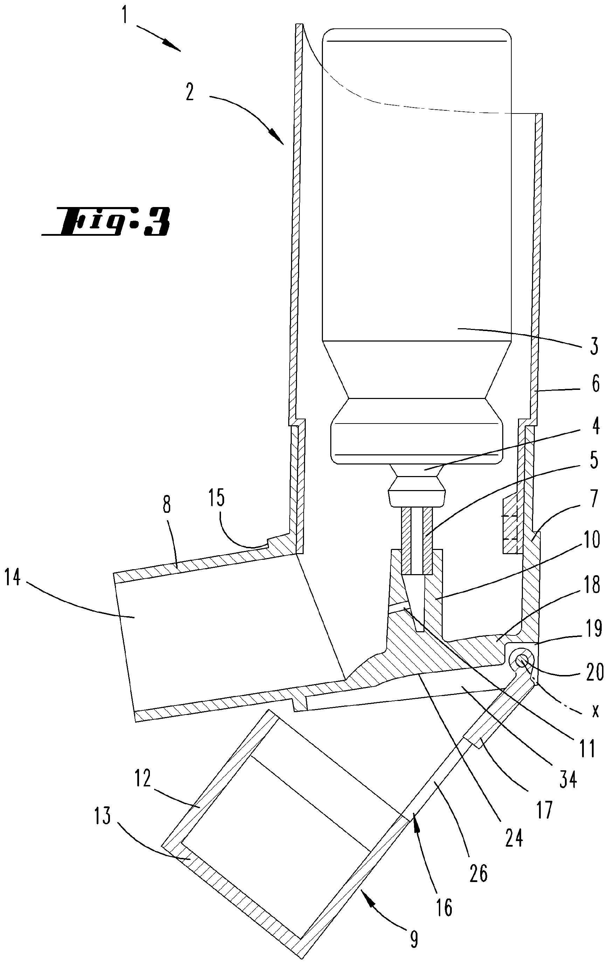

Die Kappe 9 kann hiernach mitsamt der Feder 16 und dem Anbindungsbereich 17 nach unten unter den Gehäuseboden 18 abgeschwenkt werden (vergleiche Figur 3). Mit Fortfall der Zugbelastung auf die Kappe 9 ergibt sich eine entspannte Stellung der Feder 16.

Die Schwenkachse x ist quergerichtet zu der Körpermittenachse des die Kartusche 3 umfassenden Ringteils 6 sowie quergerichtet zu einer Längsmittelachse des Mundstückes 8.

Wirkt die Feder 16 auch in der Mundstück-Verschlussstellung gemäß Figur 1 auf die Kappe 9 ein, so kann auf eine Verrastung der Kappe 9 an dem Mundstück 8 verzichtet werden.

Die vorstehenden Ausführungen dienen der Erläuterung der von der Anmeldung insgesamt erfassten Erfindungen, die den Stand der Technik zumindest durch die folgenden Merkmalskombinationen jeweils auch eigenständig weiterbilden, nämlich:
Ein Handgerät, das dadurch gekennzeichnet ist, dass die Feder 16 in einer Draufsicht, in welcher sich die Schwenkachse x als Linie abbildet, einen abgewinkelten Verlauf aufweist.

Ein Handgerät, das dadurch gekennzeichnet ist, dass die Feder 16 zwei Federarme 25, 26 aufweist, die an zwei quer zu der Öffnungsrichtung r distanzierten Befestigungsstellen 27, 28 mit der Kappe 9 verbunden sind.

Ein Handgerät, das dadurch gekennzeichnet ist, dass die Kappe 9 in einer Öffnungsrichtung r aus einer Verschlussstellung in eine Öffnungsstellung zu bewegen ist.

Ein Handgerät, das dadurch gekennzeichnet ist, dass die Feder 16 in einem entspannten Zustand über ihre Länge gesehen teilweise mit einer Richtungskomponente entgegen der Öffnungsrichtung r verläuft.

Ein Handgerät, das dadurch gekennzeichnet ist, dass die Federarme 25, 26 gehäuseseitig in einem gemeinsamen Anbindungsbereich 17 zusammengeführt sind.

Ein Handgerät, das dadurch gekennzeichnet ist, dass der Anbindungsbereich 17 mit dem Gehäuse 2 über ein Gelenk verschwenkbar verbunden ist.

Ein Handgerät, das dadurch gekennzeichnet ist, dass die Federarme 25, 26 sich in einer Fläche erstrecken, die im Verschlusszustand angepasst an eine Zuordnungsfläche 24 des Gehäuses 2 verläuft.

Ein Handgerät, das dadurch gekennzeichnet ist, dass die Kappe 9 in der Verschlussstellung mit Vorspannung an dem Gehäuse 2 anliegt.

Ein Handgerät, das dadurch gekennzeichnet ist, dass die Feder 16 in der Draufsicht W-förmig gestaltet ist, wobei ein W-Mittelbereich 29 durch den Anbindungsbereich 17 gegeben ist.

Alle offenbarten Merkmale sind (für sich, aber auch in Kombination untereinander) erfindungswesentlich. In die Offenbarung der Anmeldung wird hiermit auch der Offenbarungsinhalt der zugehörigen/beigefügten Prioritätsunterlagen (Abschrift der Voranmeldung) vollinhaltlich mit einbezogen, auch zu dem Zweck, Merkmale dieser Unterlagen in Ansprüche vorliegender Anmeldung mit aufzunehmen. Die Unteransprüche charakterisieren mit ihren Merkmalen eigenständige erfinderische Weiterbildungen des Standes der Technik, insbesondere um auf Basis dieser Ansprüche Teilanmeldungen vorzunehmen.

**Bezugszeichenliste:**

| | | | |
|---|---|---|---|
| 1 | Handgerät | 25 | Federarm |
| 2 | Gehäuse | 26 | Federarm |
| 3 | Kartusche | 27 | Befestigungsstelle |
| 4 | Kartuschenkopf | 28 | Befestigungsstelle |
| 5 | Ventilrohr | 29 | W-Mittelbereich |
| 6 | Ringteil | 30 | Federarmabschnitt |
| 7 | Ringteil | 31 | Federarmabschnitt |
| 8 | Mundstück | 32 | Umkehrbereich |
| 9 | Kappe | 33 | Umkehrbereich |
| 10 | Stützabschnitt | 34 | Gehäusewandabschnitt |
| 11 | Strömungskanal | 35 | Filmscharnier |
| 12 | Wandung | 36 | Filmscharnier |
| 13 | Kappendecke | | |
| 14 | Mundstücköffnung | | |
| 15 | Stufe | | |
| 16 | Feder | | |
| 17 | Anbindungsbereich | | |
| 18 | Gehäuseboden | r | Öffnungsrichtung |
| 19 | Vertiefung | x | Schwenkachse |
| 20 | Endabschnitt | | |
| 21 | Achse | | |
| 22 | Bohrung | | |
| 23 | | | |
| 24 | Zuordnungsfläche | | |

## Patentansprüche

1. Handgerät (1) zur Ausgabe und Inhalation einer pharmazeutischen Substanz, mit einem ein Mundstück (8) aufweisenden Gehäuse (2), wobei das Mundstück (8) mit einer Kappe (9) verschließbar ist und die Kappe (9) an dem Gehäuse (2) angebunden ist und zur Freigabe des Mundstückes (8) um eine Schwenkachse (x) verschwenkbar ist, wobei weiter die Kappe zunächst zur Freilegung des Mundstücks in einer Öffnungsrichtung im Wesentlichen linear verlagerbar ist und hiernach abschwenkbar ist, **dadurch gekennzeichnet, dass** die Kappe (9) an dem Gehäuse (2) mittels einer Feder (16) angebunden ist, dass die Feder (16) in einer Draufsicht, in welcher sich die Schwenkachse (x) als Linie abbildet, einen abgewinkelten Verlauf aufweist, dass die Kappe (9) entgegen einer Vorspannung der Feder (16) in der Öffnungsrichtung verlagerbar ist, dass die Feder (16) zwei Federarme (25, 26) aufweist, die an zwei quer zu der Öffnungsrichtung (r) distanzierten Befestigungsstellen (27, 28) mit der Kappe (9) verbunden sind, dass die Federarme (25, 26) gehäuseseitig in einem gemeinsamen Anbindungsbereich, der mit dem Gehäuse über ein Gelenk verschwenkbar verbunden ist, zusammengeführt sind und dass die Kappe (9) mitsamt dem Anbindungsbereich (17) nach unten unter einen Gehäuseboden abschwenkbar ist.

2. Handgerät nach einem der vorhergehenden Ansprüche, **dadurch gekennzeichnet, dass** die Federarme (25, 26) gehäuseseitig in einem gemeinsamen Anbindungsbereich (17) zusammengeführt sind.

3. Handgerät nach Anspruch 2, **dadurch gekennzeichnet, dass** der Anbindungsbereich (17) mit dem Gehäuse (2) über ein Gelenk verschwenkbar verbunden ist.

4. Handgerät nach einem der vorhergehenden Ansprüche, **dadurch gekennzeichnet, dass** die Federarme (25, 26) sich in einer Fläche erstrecken, die im Verschlusszustand angepasst an eine Zuordnungsfläche (24) des Gehäuses (2) verläuft.

5. Handgerät nach einem der vorhergehenden Ansprüche, **dadurch gekennzeichnet, dass** die Kappe (9) in der Verschlussstellung mit Vorspannung an dem Gehäuse (2) anliegt.

6. Handgerät nach einem der Ansprüche 2 bis 5, **dadurch gekennzeichnet, dass** die Feder (16) in der Draufsicht W-förmig gestaltet ist, wobei ein W-Mittelbereich (29) durch den Anbindungsbereich (17) gegeben ist.

## Claims

1. A handset (1) for delivering and inhaling a pharmaceutical substance, with a housing (2) that exhibits a mouthpiece (8), wherein the mouthpiece (8) can be sealed with a cap (9), and the cap (9) is attached to the housing (2), and can be swiveled around a swivel axis (x) to release the mouthpiece (8), wherein the cap can further initially be essentially linearly displaced in an opening direction to uncover the mouthpiece, and can hereby be downwardly swiveled, **characterized in that** the cap (9) is attached to the housing (2) by means of a spring (16), that the spring (16) has an angled progression in a top view, in which the swivel axis (x) is imaged as a line, that the cap (9) can be shifted in the opening direction opposite a prestress of the spring (16), that the spring (16) has two spring arms (25, 26), which are joined with the cap (9) at two fastening points (27, 28) spaced apart transversely to the opening direction (r), that the spring arms (25, 26) are brought together on the housing side in a shared attachment region (17), which is swivelably connected with the housing (2) by a hinge, and that the cap (9) together with the attachment region (17) can be downwardly swiveled under a housing floor.

2. The handset according to one of the preceding claims, **characterized in that** the spring arms (25, 26) are brought together on the housing side in a shared attachment region (17).

3. The handset according to claim 2, **characterized in that** the attachment region (17) is swivelably connected with the housing (2) by a hinge.

4. The handset according to one of the preceding claims, **characterized in that** the spring arms (25, 26) extend in a surface which, in the sealed state, runs adjusted to an allocation surface (24) of the housing (2).

5. The handset according to one of the preceding claims, **characterized in that** the cap (9) abuts against the housing (2) exposed to a prestress in the sealed position.

6. The handset according to one of claims 2 to 5, **characterized in that** the spring (16) has a W-shaped design as viewed from above, wherein a W-central region (29) is provided by the attachment region (17).

## Revendications

1. Dispositif manuel (1) pour la distribution et l'inhalation d'une substance pharmaceutique, comprenant un boîtier (2) présentant un embout buccal (8), dans lequel l'embout buccal (8) peut être fermé par un capuchon (9) et le capuchon (9) est relié au boîtier (2) et peut pivoter autour d'un axe de pivotement (x) pour libérer l'embout buccal (8), dans lequel en outre le capuchon est d'abord déplaçable de manière sensiblement linéaire dans une direction d'ouverture pour exposer l'embout buccal, puis est pivotable, **caractérisé en ce que** le capuchon (9) est relié au boîtier (2) au moyen d'un ressort (16), **en ce que** le ressort (16), en vue de dessus dans laquelle l'axe de pivotement (x) est représenté sous la forme d'une ligne, s'étend de manière coudée, **en ce que** le capuchon (9) est déplaçable dans la direction d'ouverture contre une pré-tension du ressort (16), **en ce que** le ressort (16) présente deux bras élastiques (25, 26) qui sont liés au capuchon (9) en deux points de fixation (27, 28) distants transversalement par rapport à la direction d'ouverture (r), **en ce que** les bras élastiques (25, 26) sont liés du côté du boîtier dans une zone de liaison commune qui est reliée de manière pivotante au boîtier par une articulation, et **en ce que** le capuchon (9) ensemble avec la zone de liaison (17) peut pivoter vers le bas sous une base du boîtier.

2. Dispositif manuel selon la revendication précédente, **caractérisé en ce que** les bras élastiques (25, 26) sont liés du côté du boîtier dans une zone de liaison commune (17).

3. Dispositif manuel selon la revendication 2, **caractérisé en ce que** la zone de liaison (17) est reliée de manière pivotante au boîtier (2) par une articulation.

4. Dispositif manuel selon l'une des revendications précédentes, **caractérisé en ce que** les bras élastiques (25, 26) s'étendent dans une surface qui, à l'état fermé, s'étend de manière adaptée à une surface associée (24) du boîtier (2).

5. Dispositif manuel selon l'une des revendications précédentes, **caractérisé en ce que**, dans la position fermée, le capuchon (9) repose avec précontrainte contre le boîtier (2).

6. Dispositif manuel selon l'une des revendications 2 à 5, **caractérisé en ce que** le ressort (16) est en forme de W en vue de dessus, dans lequel une zone centrale (29) du W est fournie par la zone de liaison (17).
